# EUROPEAN PATENT SPECIFICATION

(11) **EP 1 536 742 B1**
(45) Date of publication and mention of the grant of the patent: **02.11.2006**
(21) Application number: 03750870.2
(22) Date of filing: 07.08.2003
(51) Int. Cl.: A61F 2/46

(54) **AN INSTRUMENT FOR PREPARING A BONE CEMENT MATERIAL**
EIN INSTRUMENT ZUM VORBEREITEN VON KNOCHENZEMENTMATERIAL
INSTRUMENT POUR PREPARER UN MATERIAU CIMENTAIRE OSSEUX

(30) Priority: 07.08.2002 GB 0218310
(43) Date of publication of application: 08.06.2005
(73) Proprietor: Depuy International Limited, Beeston, Leeds LS11 8DT (GB)
(72) Inventor: WATKINS, Neil, David, Lymm Cheshire WA13 0SR (GB); ISAAC, Graham, Honley Huddersfield HD9 6QX (GB)
(74) Representative: Belcher, Simon James
(86) International application number: PCT/GB2003/003443
(87) International publication number: WO 2004/014262

(56) References cited:
- EP-A- 0 444 842
- EP-A- 0 872 223
- WO-A-96/38104
- WO-A-97/21485
- DE-A- 3 835 853
- DE-A- 19 848 479
- FR-A- 2 629 337
- US-A- 5 591 171
- US-A- 6 086 594

## Description

This invention relates to an instrument for preparing and delivering a bone cement material for deployment in orthopaedic surgery.

It is common to use bone cement materials to fix orthopaedic implants such as components of orthopaedic joint prostheses. The materials are commonly based on methacrylic acid and its esters. The materials are prepared by mixing two reactive components together. Reaction of the components involves polymerisation of the acid.

The reliability of the bond between a bone cement and adjacent bone tissue requires that the cement be injected into a bone cavity under pressure. This can improve the keying of the cement into the surface of the bone. It can also reduce the tendency for blood to exude from the surface of the bone, which itself can otherwise lead to weakening of the bond between the cement and the bone tissue, and weakening of the cement itself. A cement injection pressure of not less than about 3 kPa can be sufficient to prevent exudation of blood from bone tissue. It has been established through cadaveric studies that an injection pressure of at least about 250 kPa can lead to impregnation of porous bone tissue by bone cement, for example to a depth of about 3 to 4 mm.

DE-19848479 discloses an instrument for filling an opening in a bone with bone cement. The instrument delivers bone cement through a feed line at a constant volume flow rate. The flow is stopped when the pressure in the bone cement reaches a value of between 26,7 kPa and 400 kPa (200 and 3000 mm Hg). The bone cement feed has a control to ensure that the bone cement is pressed through the feed line at a constant volume flow rate. A switch stops the flow when the pressure of the bone cement reaches a set level at the supply vessel and/or in the feed line.

The invention provides an instrument for deploying a bone cement material in a bone cavity, the material being formed from two reactive components which, when mixed, react to form a useable cement, the instrument comprising:
a. a chamber in which the components of the material can be mixed,
b. a mixing tool which extends into the chamber, and which can be manipulated from outside the chamber, to cause the components of the material to mix,
c. an outlet from the chamber through which the mixed material can be discharged from the chamber after it has mixed,
d. a piston which can be moved through the chamber to apply positive pressure to mixed material within the chamber, to displace the mixed material from the chamber through the outlet into the bone cavity,
e. a sealing component which fits over the bone cavity to seal the cavity around the chamber outlet and to minimise leakage of bone cement that has been injected into the cavity, and
f. a sensor for measuring the pressure to which the bone cement is subjected during displacement from the chamber, in which the sensor is located in the sealing component.

The instrument of the invention has the advantage that it enables greater accuracy to be achieved in the application of the pressure to cement which is injected into a bone cavity. This can help to optimise the bond between the cement and the surface of the bone. It can also help to minimise the tendency for blood to exude from the bone at the surface where it contacts the cement.

The instrument of the invention has the further advantage that mixing of the cement material and its subsequent deployment can be accomplished, with control over the pressure that is applied to the cement, while the material is retained within the chamber and without exposure to atmosphere. This reduces the risk of contamination of the material, and also reduces the risk of exposure to the possibly harmful components of the cement.

Preferably, a further pressure sensor is located in at least one of:
a. the wall of the chamber towards which the piston moves to discharge mixed cement material through the chamber outlet,
b. the face of the piston which faces towards the end wall of the chamber, and
c. the outlet from the chamber

Preferably, the further pressure sensor is located in or close to the face of the sealing component which faces into the bone cavity. This allows pressure signals to be generated which most accurately represent the pressure to which bone cement within the cavity is subjected, allowing accurate control over the interface between the cement and the surface of bone tissue.

Preferably, the sealing component comprises:
a. a sealing plate which can be fitted over the bone cavity and has a quantity of a resiliently deformable material on its lower face to enable a seal to be created between the plate and the edge of the bone, and
b. a plug which has an injection port extending through it in which the outlet can be received,
the sealing plate having an opening extending through it (i) in which the plug can be received, and (ii) through which a prosthesis which is to be bonded to the bone of the cavity by the bone cement can be inserted into the cavity after injection of the cement and removal of the plug.

When the sealing component comprises a sealing plate and a plug, it can be preferred for the pressure sensor to be located in the sealing plate.

Preferably, the pressure sensor generates a signal which is representative of the pressure to which the bone cement is subjected during displacement from the chamber, and in which the signal gives rise to an indication of the said pressure which is audible or visible. For example, the pressure sensor can generate a signal which is indicated on a pressure scale. The pressure sensor can generate a signal which is indicated on one or more lights; for example different coloured lights can be illuminated according to the pressure to which the cement is subjected. One arrangement might provide one or more lights which can be differentiated, for example by position or colour, which are illuminated when the pressure to which the cement is subjected is within a predetermined range, and one or more other lights which are illuminated when the pressure to which the cement is subjected is outside a predetermined range, for example above the range or below the range or both.

Preferably, the pressure signal causes a warning signal to be generated when the pressure to which the bone cement is subjected during displacement from the chamber is less than about 3 kPa. More preferably, the pressure sensor causes a warning signal to be generated when the pressure to which the bone cement is subjected during displacement from the chamber is less than about 150 kPa, especially less than about 200 kPa, for example less than about 250 kPa. A warning signal can be generated when the pressure exceeds about 450 kPa, preferably about 400 kPa, more preferably about 350 kPa.

Preferably, the pressure sensor generates a signal which is representative of the pressure to which the bone cement is subjected during displacement from the chamber, and in which the signal can be arranged to cause the pressure that is applied to the bone cement by the piston to be changed when the pressure is outside a predetermined range. The lower limit of the said predetermined pressure range is preferably at least about 3 kPa. More preferably, the lower limit is at least about 150 kPa, especially at least about 200 kPa, for example at least about 250 kPa. Preferably, the upper limit of the said pressure range is about 650 kPa, more preferably about 500 kPa, especially about 400 kPa.

Mixing devices for bone cement, which include discharge outlets for the cement after it has been mixed, are known. These include devices in which the piston which is used to discharge cement from the device forms one end of the chamber in which the cement is mixed. In some such devices, the discharge outlet is used during mixing of the cement to move the tool, for example by means of a shaft which protrudes from the outlet.

The mixing tool can move within the chamber to ensure mixing of the cement components. Preferably, the tool is capable of rotating about an axis defined by a shaft thereof, and is also capable of moving inwardly and outwardly along the axis defined by the shaft. These movements can be made manually, or using a motor or other drive. The mixing tool can have vanes on it. The mixing tool and the pressure sensor have to be positioned so that neither of them is damaged when the mixing tool is moved to cause the bone cement components to mix.

Especially when the pressure sensor is provided in a wall of the chamber or in the piston, it can be preferred for it to be movable between a retracted or withdrawn position in which it allows the mixing tool to be used to mix the components, and an operative position in which it extends further into the chamber, into bone cement material in the chamber which has been mixed and is being allowed to react. The pressure sensor can be separable from the chamber so that they are supplied as separate parts. The pressure sensor can then be inserted into the chamber wall after the mixing step has been completed. For example, the chamber wall can have an opening which is closed by a plug or a cap or other closure. The pressure sensor can be positioned in the opening after removal of the plug.

The pressure sensor can provide an accurate indication of the pressure to which cement being discharged from the chamber is exposed when it is provided in a wall of the chamber (including the outlet, which might be a nozzle, from the chamber) or in the piston. For example, the sensor can be positioned in the end wall of the chamber, towards which the piston moves to displace cement from the chamber, or it can be positioned in the outlet through which cement is injected into a bone cavity.

It can be preferred for the step of preparing the instrument of the invention for use (possibly as part of a manufacturing process) to include a step of calibrating the pressure sensor, especially when the sensor is provided in a wall of the chamber or in the piston.

The pressure sensor can comprise at least one pressure switch or pressure transducer. Pressure might also be indicated by means of a component which changes configuration or shape or location when the pressure that is sensed by the sensor exceeds a pre-set limit.
For example, it might comprise a pop-out plug which remains in place in a socket until the pressure that is sensed by the sensor reaches the pre-set limit, and then reverts to a popped configuration, providing a visible indication of pressure being above the said limit. The socket can have a bleed hole in its base which communications with the space in which the pressurised bone cement is located.. The plug will be sealed into the socket, for example by a flexible polymeric seal which allows the plug to flex between its configuration prior to use, and its popped configuration.

Preferably, the instrument includes a data processor which is connected to the pressure sensor so that data from the pressure sensor can be transmitted to the data processor for analysis, to generate information concerning the pressure of material within the chamber. The information can be transmitted to the data processor wirelessly, for example, using radio or infrared communication. Generally, however, it will be preferred for the information to be transmitted using conductors.

Use of a data processor has the advantage that it can provide a record of the measurements taken from the cement material in the chamber, which can be retained for later reference, for example with other records relating to the procedure. A data processor can be used to generate output information, for example for use by the surgeon. This information can be visual information, especially in graphical form. The information could be audible.

Embodiments of the present invention will now be described by way of example with reference to the accompanying drawings, in which:
Figure 1 is a schematic side view of an instrument according to the invention.
Figure 2 is an enlarged view of a seal component of the instrument according to the invention.
Figure 3 is a schematic view showing steps in a method of using the instrument of the invention.
Figure 4 shows another embodiment of seal component of the instrument according to the invention.

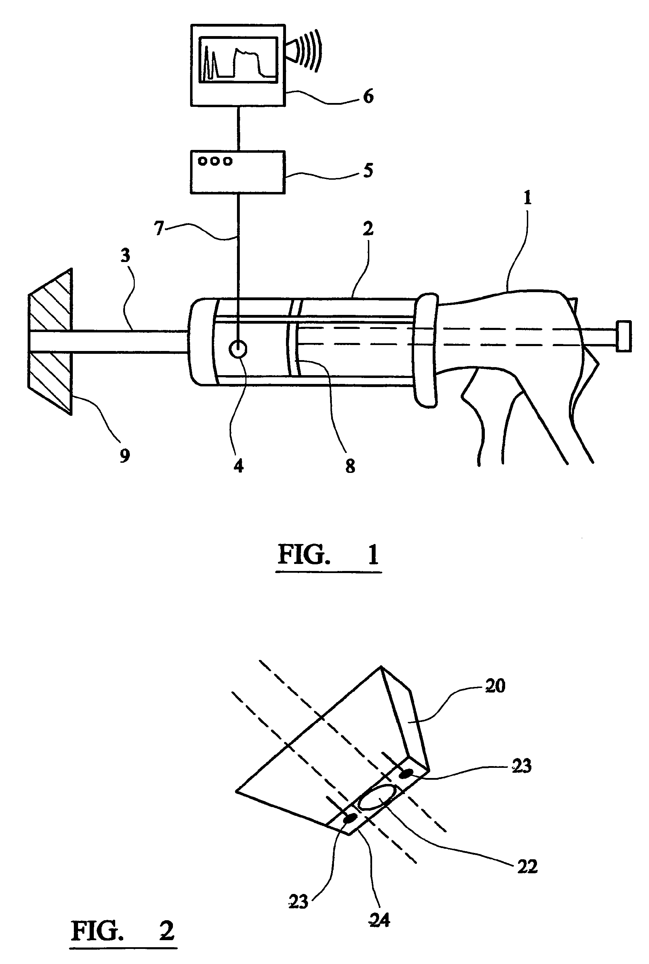

Referring to the drawings, Figure 1 shows an instrument 1 for deploying a bone cement material in a bone cavity. Bone cement materials are known which can be mixed and deployed from an instrument of this general kind, for example as sold by DePuy CMW. They are formed from two reactive components which, when mixed, react to form a useable cement.

The instrument shown in Figure 1 comprises a chamber 2 in which the components of the material can be mixed. The chamber can contain a mixing tool which can be manipulated from outside the chamber, to cause the components of the material to mix. Chamber has an outlet 3 in the form of a nozzle, through which the mixed material can be discharged from the chamber after it has mixed. The material is displaced from the chamber 2 through the outlet 3 by means of a piston 8.

The instrument includes a sealing component 9 which fits over the bone cavity to seal the cavity around the chamber outlet and to minimise leakage of bone cement that has been injected into the cavity.

A sensor 4 is provided in a wall of the chamber for measuring the pressure to which the bone cement is subjected during displacement from the chamber. A pressure signal which is generated at the sensor is conducted by a signal cable 7 to a data processor, and then to a display device, which can provide for example a visual, graphical or audible signal output.

Figure 2 shows a seal component 20 which is configured to fit into a cavity in a bone to seal the bone against egress of cement during injection. The component is therefore shaped according to the anticipated shape of the cavity in the bone.

The sealing component has a bore 22 extending through it into which the outlet nozzle 3 of the instrument can be inserted. Two pressure transducers 22 are mounted within the seal component on the face 24 thereof which faces into the cavity in the bone when the component is in use, so that they can sense accurately the pressure within the cavity.

Figure 3a shows the instrument 1 of the invention being offered up to a prepared femur 30, with the nozzle 3 introduced into the cavity 32 in the bone.

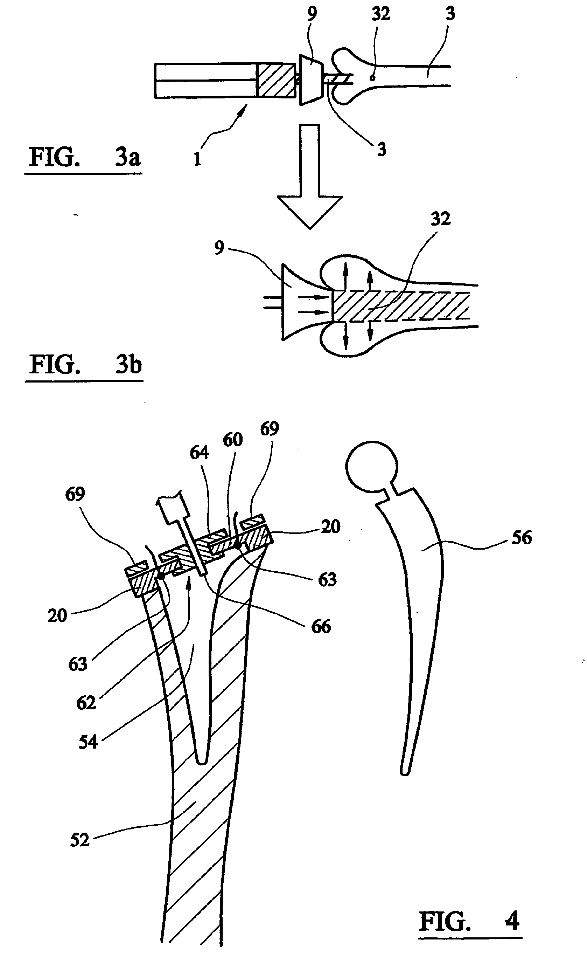

Figure 3b shows the instrument in position relative to the bone during the injection of bone cement into the cavity. The sealing component 9 is in contact with the bone so as to form a seal between it and the bone, so that loss of cement from the bone cavity is minimised during the injection.

Figure 4 shows a femur 52 which has been resected at its upper end. Cancellous bone has been removed from the centre of the femur at the upper end to form a cavity 54 in which a prosthesis 56 is to be located. The prosthesis comprises a stem which is received in the cavity and a head which engages an acetabular cup component, as is known.

The stem of the prosthesis is bonded to the bone of the cavity by means of bone cement injected into the cavity under pressure. Pressure in the cement injected into the cavity is maintained during and after injection (while the cement hardens) by means of a sealing gasket positioned over the end of the bone. The gasket comprises a sealing plate 60 having an opening 62 in it, and a plug 64 located in the opening in which the nozzle 66 of bone cement delivery apparatus can be received.

Pressure sensors 63 are provided in the sealing plate which can generate a signal which represents the pressure within the cavity in the bone. The sensors can indicate the pressure with which cement is injected into the cavity from within the chamber 2 of the instrument.

Force can be applied to the gasket against the bone directly, or by means of a u-shaped tool 69 which can be located around the opening 62 in the sealing plate.

The dimensions of the sealing plate will be selected according to the bone against which it is to be used and the prosthesis which is to be inserted into the bone cavity. For example, a sealing plate to be used against the femur of an adult human might have dimensions 60 mm by 55 mm. The opening in the plate has dimensions 20 mm by 11 mm.

The opening 62 in the sealing plate is generally oval, having semi-circular portions at each end joined by straight portions.

In use, after injection of bone cement into the bone cavity, the plug 64 is removed from the opening 62 in the sealing plate 60. The plug can be removed soon after injection of the cement. However, it can be preferred for its removal to be deferred until the cement has partially hardened, for example, until it has hardened sufficiently for insertion into the cavity of the prosthesis. The prosthesis to be located in the bone cavity can be inserted in the cavity through the opening 62 after removal of the plug. The pressure sensors can provide an indication of the pressure that is generated within the bone cavity during the step of inserting the prosthesis into the cavity.

Once the bone cement has hardened sufficiently to bond the prosthesis to the bone, the sealing plate can be removed from the bone. Preferably, the sealing plate is severed before removal, for example by tearing or more preferably by cutting, from the opening 62 to an edge of the plate, so that it can be removed by lateral movement rather than having to be passed over the end of the prosthesis.

Further details of a sealing component of the type shown in Figure 4 are disclosed in US-5980527.

## Claims

1. An instrument (1) for deploying a bone cement material in a bone cavity, the material being formed from two reactive components which, when mixed, react to form a useable cement, the instrument comprising:
a. a chamber (2) in which the components of the material can be mixed,
b. a mixing tool which extends into the chamber (2), and which can be manipulated from outside the chamber (2), to cause the components of the material to mix,
c. an outlet (3) from the chamber (2) through which the mixed material can be discharged from the chamber (2) after it has mixed,
d. a piston (8) which can be moved through the chamber (2) to apply positive pressure to mixed material within the chamber (2), to displace the mixed material from the chamber (2) through the outlet (3) into the bone cavity (32), and
e. a sealing component (9) which fits over the bone cavity (32) to seal the cavity around the chamber outlet (3) and to minimise leakage of bone cement that has been injected into the cavity, **characterised in that** the instrument further comprises:
f. a sensor (4) for measuring the pressure to which the bone cement is subjected during displacement from the chamber (2), in which the sensor (4) is located in the sealing component (9).

2. An instrument (1) as claimed in claim 1, which includes a further pressure sensor (4) which is located in at least one of:
a. the wall of the chamber (2) towards which the piston (8) moves to discharge mixed cement material through the chamber outlet (3),
b. the face of the piston (8) which faces towards the end wall of the chamber (2), and
c. the outlet (3) from the chamber (2).

3. An instrument (1) as claimed in claim 2, in which the further pressure sensor (4) is located in or close to the face of the sealing component (9) which faces into the bone cavity (32).

4. An instrument (1) as claimed in claim 1, in which the sealing component (9) comprises:
a. a scaling plate (60) which can be fitted over the bone cavity (32) and has a quantity of a resiliently deformable material on its lower face to enable a seal to be created between the plate (60) and the edge of the bone, and
b. a plug (64) which has an injection port extending through it in which the outlet can be received.
the sealing plate (60) having an opening (62) extending through it (i) in which the plug (64) which can be received, and (ii) through which a prosthesis which is to be bonded to the bonc of the cavity (32) by the bone cement can be inserted into the cavity (32) after injection of the cement and removal of the plug (64).

5. An instrument (1) as claimed in claim 4, in which the pressure sensor (4) is located in the sealing plate (60).

6. An instrument (1) as claimed in claim 1, in which the pressure sensor (4) generates a signal which is representative of the pressure to which the bone cement is subjected during displacement from the chamber (2), and in which the signal gives rise to an indication of the said pressure which is audible or visible.

7. An instrument (1) as claimed in claim 6, in which the pressure sensor (4) causes a warning signal to be generated when the pressure to which the bone cement is subjected during displacement from the chamber (2) is less than about 3 kPa.

8. An instrument (1) as claimed in claim 1, in which the pressure sensor (4) generates a signal which is representative of the pressure to which the bone cement is subjected during displacement from the chamber (2), and in which the signal can be arranged to cause the pressure that is applied to the bone cement by the piston (8) to be changed when the pressure is outside a pre-determined range.

## Patentansprüche

1. Instrument (1) zum Einbringen eines Knochenzementmaterials in einen Knochenhohlraum, wobei das Material aus zwei reaktiven Komponenten gebildet ist, die, wenn sie gemischt werden, reagieren, um einen verwendbaren Zement zu bilden, wobei das Instrument aufweist:
a. eine Kammer (2), in der die Komponenten des Materials gemischt werden können,
b. ein Mischwerkzeug, das sich in die Kammer (2) erstreckt und das von außerhalb der Kammer (2) gehandhabt werden kann, um zu bewirken, daß sich die Komponenten des Materials mischen,
c. einen Auslaß (3) aus der Kammer (2), durch den das gemischte Material aus der Kammer (2) ausgegeben werden kann, nachdem es sich vermischt hat,
d. einen Kolben (8), der durch die Kammer (2) bewegt werden kann, um positiven Druck auf gemischtes Material innerhalb der Kammer (2) aufzubringen, um das gemischte Material aus der Kammer (2) durch den Auslaß (3) in den Knochenhohlraum (32) zu verschieben, und
e. eine Dichtkomponente (9), welche über den Knochenhohlraum (32) paßt, um den Hohlraum um den Kammerauslaß (3) abzudichten und um das Auslaufen von Knochenzement, der in den Hohlraum eingespritzt worden ist, zu minimieren, **dadurch gekennzeichnet, daß** das Instrument weiter aufweist:
f. einen Sensor (4) zum Messen des Druckes, dem der Knochenzement während des Verschiebens aus der Kammer (2) ausgesetzt ist, wobei der Sensor (4) in der Dichtkomponente (9) angeordnet ist.

2. Instrument (1) nach Anspruch 1, welches einen weiteren Drucksensor (4) umfaßt, der in wenigstens einem aus:
a. der Wand der Kammer (2), in Richtung auf die sich der Kolben (8) bewegt, um gemischtes Zementmaterial durch den Kammerauslaß (3) auszugeben,
b. der Fläche des Kolbens (8), der der Endwand der Kammer (2) zugewandt ist, und
c. dem Auslaß (3) aus der Kammer (2)
angeordnet ist.

3. Instrument (1) nach Anspruch 2, bei dem der weitere Drucksensor (4) in oder nahe der Fläche der Dichtkomponente (9) angeordnet ist, die in den Knochenhohlraum (32) weist.

4. Instrument (1) nach Anspruch 1, bei dem die Dichtkomponente (9) aufweist:
a. eine Dichtplatte (60), die über den Knochenhohlraum (32) gepaßt werden kann und eine Menge aus nachgiebig deformierbarem Material auf ihrer unteren Fläche hat, um zu ermöglichen, daß eine Dichtung zwischen der Platte (60) und der Kante des Knochens erzeugt wird, und
b. einen Stopfen (64), welcher eine Einspritzöffnung hat, die sich durch ihn hindurch erstreckt, in der der Auslaß aufgenommen werden kann;
wobei die Dichtplatte (60) eine Öffnung (62) aufweist, die sich durch sie erstreckt, (i), in der der Stopfen (64) aufgenommen werden kann, und (ii) durch den eine Prothese, die mit dem Knochen des Hohlraums (42) durch den Knochenzement verbunden werden soll, in den Hohlraum (32) nach dem Einspritzen des Zements und dem Entfernen des Stopfens (24) eingesetzt werden kann.

5. Instrument (1) nach Anspruch 4, bei dem sich der Drucksensor (4) in der Dichtplatte (60) befindet.

6. Instrument (1) nach Anspruch 1, bei dem der Drucksensor (4) ein Signal erzeugt, das repräsentativ für den Druck ist, dem der Knochenzement während des Verschiebens aus der Kammer (2) ausgesetzt ist, und bei dem das Signal eine Angabe des Druckes ausgibt, die hörbar oder sichtbar ist.

7. Instrument (1) nach Anspruch 6, bei dem der Drucksensor (4) bewirkt, daß ein Warnsignal erzeugt wird, wenn der Druck, dem der Knochenzement während des Verschiebens aus der Kammer (2) ausgesetzt ist, geringer ist als ungefähr 3 kPa.

8. Instrument (1) nach Anspruch 1, bei dem der Drucksensor (4) ein Signal erzeugt, das repräsentativ für den Druck ist, dem der Knochenzement während des Verschiebens aus der Kammer (2) ausgesetzt ist, und bei dem das Signal so eingerichtet werden kann, zu bewirken, daß der Druck, der von dem Kolben (8) auf den Knochenzement ausgeübt wird, geändert wird, wenn der Druck außerhalb eines vorbestimmten Bereiches liegt.

## Revendications

1. Un instrument (1) pour déployer un matériau cimentaire osseux dans une cavité osseuse, le matériau étant formé à partir de deux composants réactifs qui, une fois mélangés, réagissent pour former un ciment utilisable, l'instrument comprenant :
a. une chambre (2), dans laquelle les composants du matériau peuvent être mélangés,
b. un outil de mélange, qui s'étend dans la chambre (2) et qui peut être manipulé depuis l'extérieur de la chambre (2), pour mélanger les composants du matériau,
c. une sortie (3) partant de la chambre (2), à travers laquelle le matériau mélangé peut être déchargé de la chambre (2) après avoir été mélangé,
d. un piston (8) pouvant être déplacé à travers la chambre (2) pour appliquer une pression positive au matériau mélangé présent dans la chambre (2), pour déplacer le matériau mélangé de la chambre (2) à travers la sortie (3) et faire pénétrer dans la cavité osseuse (32), et
e. un composant d'étanchéité (9) qui se monte sur la cavité osseuse (32) pour fermer hermétiquement la cavité autour de la sorte de chambre (3) et minimiser les fuites de ciment osseux ayant été injecté à l'intérieur de la cavité, **caractérisé en ce que** l'instrument comprend en outre :
f. un capteur (4) pour mesurer la pression à laquelle le ciment osseux est soumis durant le déplacement depuis la chambre (2), dans lequel le capteur (4) est placé dans le composant d'étanchéité (9).

2. Un instrument (1) tel que revendiqué à la revendication 1, comprenant un autre capteur de pression (4), placé dans au moins l'une parmi :
a. la paroi de la chambre (2) vers laquelle le piston (8) se déplace pour décharger du matériau cimentaire mélangé à travers la sortie de chambre (3),
b. la face du piston (8) tournée vers la paroi d'extrémité de la chambre (2), et
c. la sortie (3) de la chambre (2).

3. Un instrument (1) tel que revendiqué à la revendication 2, dans lequel l'autre capteur de pression (4) est placé dans ou à proximité de la face du composant d'étanchéité (9) tournée dans la cavité osseuse (32).

4. Un instrument (1) tel que revendiqué à la revendication 1, dans lequel le composant d'étanchéité (9) comprend :
a. une plaque d'étanchéité (60), pouvant être montée sur la cavité osseuse (32) et ayant une quantité de matériau déformable élastiquement sur sa face inférieure, afin de permettre de créer un joint d'étanchéité entre la plaque (60) et le bord de l'os, et
b. un bouchon (64) ayant un orifice d'injection s'étendant à travers lui, dans lequel la sortie peut être logée,
la plaque d'étanchéité (60) ayant une ouverture (62) s'étendant à travers elle, (i) dans laquelle le bouchon (64) peut être logé, et (ii) à travers laquelle une prothèse qui doit être reliée à l'os de la cavité (32) par le ciment osseux peut être insérée dans la cavité (32), après injection du ciment et enlèvement du bouchon (64).

5. Un instrument (1) tel que revendiqué à la revendication 4, dans lequel le capteur de pression (4) est placé dans la plaque d'étanchéité (60).

6. Un instrument (1) tel que revendiqué dans la revendication 1, dans lequel le capteur de pression (4) génère un signal représentatif de la pression à laquelle le ciment osseux est soumis durant le déplacement depuis la chambre (2), et dans lequel le signal donne lieu à une indication de ladite pression, qui est audible ou visible.

7. Un instrument (1) tel que revendiqué à la revendication 6, dans lequel le capteur de pression (4) provoque un signal d'avertissement devant être généré lorsque la pression à laquelle le ciment osseux est soumis pendant le déplacement depuis la chambre (2) est inférieure à environ 3 kPa.

8. Un instrument (1) tel que revendiqué à la revendication 1, dans lequel le capteur de pression (4) génère un signal représentatif de la pression à laquelle le ciment osseux est soumis durant le déplacement depuis la chambre (2), et dans lequel le signal peut être agencé pour que la pression, qui est appliquée sur le ciment osseux par le piston (8), soit modifiée lorsque la pression est hors des limites d'une fourchette prédéterminée.
